# FASCICULE DE BREVET EUROPEEN

(11) **EP 4 147 244 B1**
(45) Date de publication et mention de la délivrance du brevet: **28.01.2026**
(21) Numéro de dépôt: 21732432.6
(22) Date de dépôt: 06.05.2021
(51) Int. Cl.: G16H 10/60, G07C 9/26, G16H 50/80, G16H 40/63, G07C 9/20, A61B 5/145, A61B 5/01, A61B 5/00, G07C 9/00, G07C 9/37

(54) **DISPOSITIF D'ACCES PORTABLE ET MONO-USAGE A UNE ZONE A ACCES RESTREINT, SYSTEME DE GENERATION DE CLE D'ACCES ET PROCEDE DE CONTROLE D'ACCES ASSOCIES**
TRAGBARE EINWEGVORRICHTUNG ZUM ZUGRIFF AUF EINEN BEREICH MIT EINGESCHRÄNKTEM ZUGANG, ZUGEHÖRIGES ZUGANGSSCHLÜSSELERZEUGUNGSSYSTEM UND ZUGEHÖRIGES ZUGANGSSTEUERUNGSVERFAHREN
PORTABLE AND SINGLE-USE DEVICE FOR ACCESSING AN AREA WITH RESTRICTED ACCESS, ASSOCIATED ACCESS KEY GENERATION SYSTEM AND ASSOCIATED ACCESS CONTROL METHOD

(30) Priorité: 06.05.2020 FR 2004576
(43) Date de publication de la demande: 15.03.2023
(73) Titulaire: Grapheal, 38000 Grenoble (FR)
(72) Inventeur: DJOHARIAN, Behnaz, 38330 Biviers (FR); BOUCHIAT, Vincent, 38330 Biviers (FR)
(74) Mandataire: Santarelli
(86) Numéro de dépôt international: PCT/FR2021/050778
(87) Numéro de publication internationale: WO 2021/224578

(56) Documents cités:
- US-A- 4 621 643
- US-A- 5 209 230
- US-A1- 2003 225 324
- US-A1- 2008 295 152
- AMAY J. BANDODKAR ET AL: "Battery-free, skin-interfaced microfluidic/electronic systems for simultaneous electrochemical, colorimetric, and volumetric analysis of sweat", SCIENCE ADVANCES, vol. 5, no. 1, 18 January 2019 (2019-01-18), US, pages eaav3294, XP055754144, ISSN: 2375-2548, DOI: 10.1126/sciadv.aav3294
- KIM JAYOUNG ET AL: "Wearable biosensors for healthcare monitoring", NATURE BIOTECHNOLOGY, GALE GROUP INC, NEW YORK, vol. 37, no. 4, 25 February 2019 (2019-02-25), pages 389 - 406, XP036746307, ISSN: 1087-0156, [retrieved on 20190225], DOI: 10.1038/S41587-019-0045-Y

## Description

### DOMAINE TECHNIQUE

La présente invention concerne un dispositif d'accès portable et mono-usage à une zone à accès restreint. L'invention concerne également un système de génération de clé d'accès comprenant un tel dispositif d'accès, et un procédé de contrôle d'accès à une zone à accès restreint.

L'invention s'applique au domaine de la mesure de paramètres de santé de sujets humains ou animaux, notamment à l'analyse d'échantillons biologiques, en particulier à l'analyse d'échantillons biologiques issus de personnes ou d'animaux susceptibles d'être porteurs d'un agent pathogène, en vue d'autoriser ou non leur accès à une zone à accès restreint pour laquelle on cherche à limiter le risque de contamination.

### ÉTAT DE LA TECHNIQUE ANTÉRIEURE

Il est connu de chercher à déterminer si les conditions de santé d'un sujet, c'est-à-dire un organisme vivant tel qu'une personne ou un animal, sont compatibles avec son entrée dans une zone à accès restreint. Une telle évaluation trouve, par exemple, son sens dans le contrôle des épidémies infectieuses, celles-ci étant susceptibles d'aboutir à de véritables catastrophes sanitaires, humanitaires et économiques, parfois à l'échelle mondiale, et dont les effets délétères sont susceptibles de s'inscrire sur le long terme. L'épidémie du COVID-19 en est un parfait exemple.

Les solutions actuelles consistent généralement à tester un échantillon biologique issu du sujet dont on veut déterminer s'il peut ou non accéder à la zone à accès restreint. Alternativement ou conjointement, un tel test est réalisé directement sur le sujet.

Par « accès restreint », il est entendu une zone dont l'accès est strictement réservé à une certaine catégorie de personnes ou d'animaux, ou une zone pour laquelle on souhaite contrôler le flux d'entrée.

Par « paramètre de santé », il est entendu, au sens de la présente invention, paramètre biologique, physiologique ou chimique permettant de qualifier et/ou de quantifier un état associé à la santé d'une personne ou d'un animal.

Par « échantillon biologique », il est entendu, au sens de la présente invention, un échantillon, liquide, gazeux ou solide, prélevé sur un sujet, et dont on veut déterminer s'il comprend au moins un analyte prédéterminé. L'échantillon biologique est susceptible de provenir de n'importe quelle source biologique, comme un liquide physiologique, y compris le sang, la salive, le liquide oculaire, le liquide céphalorachidien, la sueur, l'urine, les selles, le sperme, le lait, le liquide d'ascite, les muqueuses, le liquide synovial, le liquide péritonéal, le liquide amniotique, les tissus, les cellules cultivées ou autres.

Un matériau biologique prélevé est susceptible d'être utilisé en tant qu'échantillon biologique directement tel qu'il est obtenu lors de son prélèvement, ou encore à l'issue d'un prétraitement pour en modifier le caractère. Par exemple, un matériau biologique initialement solide ou semi-solide peut être rendu liquide en le dissolvant ou en le mettant en suspension dans un milieu liquide approprié.

Par « analyte », il est entendu, au sens de la présente invention, un composé d'origine biologique qui peut être un acide nucléique ou une protéine issu(e) notamment d'un microorganisme contaminant, éventuellement infectieux, pour laquelle on cherche à déterminer la présence dans l'échantillon biologique et/ou la concentration. En particulier, la présente invention peut être très utile pour détecter un brin d'ADN (acide désoxyribonucléique) ou d'ARN (acide ribonucléique), ou une protéine spécifique des bactéries, virus, champignons et parasites impliqués dans la contamination infectieuse, parmi lesquels on peut citer à titre d'exemples la famille des coronavirus du SRAS (syndrome respiratoire aigu sévère), VIH (virus de l'immunodéficience humaine), grippe, virus Ebola, Dengue, Chikungunya ou Zika. En outre, d'autres types d'analytes peuvent être une hormone, une enzyme, le glucose, un composés chimique exogène (éthanol, composés pharmaceutiques ou stupéfiants et/ou leurs métabolites), les paramètres de coagulation (notamment les facteurs VII, V, X, II et le fibrinogène qui sont présents dans le plasma sanguin), les protéines endogènes (par exemple les protéines libérées par le muscle cardiaque), les métabolites, les acides nucléiques, etc. pour lesquels on cherche à déterminer la présence dans l'échantillon biologique et/ou la concentration.

En outre, de tels paramètres biologiques, physiologiques ou physiques sont, par exemple, une température corporelle, une tension artérielle, une fréquence cardiaque, un taux d'oxygène sanguin, un pH, etc.

De façon classique, il est connu de prélever un échantillon biologique et de l'analyser en laboratoire. Par la suite, un(e) biologiste établit un compte-rendu d'analyse, et le sujet ayant subi le prélèvement est autorisé ou non à se rendre dans la zone à accès limité suivant le contenu du compte-rendu.

Néanmoins, une telle procédure ne donne pas entière satisfaction.

En effet, une telle procédure peut difficilement être automatisée, et prend du temps, notamment lié à la logistique associée à la manutention des échantillons prélevés, de sorte qu'elle ne peut pas être appliquée, par exemple, à l'entrée d'un moyen de transport confiné (aéronef, train, navire, etc.) pour déterminer si chaque personne ayant réservé un billet d'avion ou de train peut ou non embarquer, suivant son état de santé ou son niveau de contamination. Or, de tels moyens de transport sont particulièrement sensibles aux effets de l'état de santé induit par les visiteurs ou par les employés, notamment parce qu'ils peuvent, soit mettre en péril la sécurité des usagers, soit induire des contaminations.

De la même façon, une telle procédure ne peut pas être appliquée à grande échelle, par exemple pour tester les potentiels participants et le personnel assistant à un rassemblement sportif, culturel ou religieux, ou encore pour filtrer les visiteurs ou le personnel d'un établissement social, médicosocial ou de santé, par exemple un établissement d'hébergement pour personnes âgées dépendantes (également désigné par le sigle EHPAD).

Les raisons limitant l'usage actuel sont notamment les temps minimums pour obtenir le résultat du test (généralement supérieurs à la dizaine de minutes), la fiabilité modérée des tests (existence d'un taux non négligeable de faux négatifs) et la difficile traçabilité des échantillons pouvant donner lieu à des retards, des erreurs ou des fraudes.

Un but de l'invention est donc de proposer un dispositif d'accès permettant, de façon simple, rapide et automatisée, de générer une clé d'accès permettant le filtrage de sujets suivant leur état de santé. Document US 2008/295152 A1 divulgue un dispositif d'accès à une zone à accès restreint, le dispositif comprenant un support, un capteur et un étage de contrôle d'accès. Le dispositif du US 2008/295152 A1 n'est ni portable ni mono-usage, et le support du dispositif ne comprend pas de partie jetable et de partie conservable.

### EXPOSÉ DE L'INVENTION

L'étendue de la invention est conférée par les revendications 1 à 16. cet effet, l'invention a pour objet un dispositif d'accès du type précité, dans lequel comprenant un support, un capteur et un étage de contrôle d'accès, le support comprenant une partie jetable et une partie conservable, le capteur étant porté par la partie jetable du support et étant configuré pour mesurer au moins un paramètre de santé d'un sujet, et pour délivrer un signal de détection représentatif de la mesure de chaque paramètre de santé, l'étage de contrôle d'accès étant porté par la partie conservable du support et étant configuré pour stocker, de préférence sous forme cryptée, des informations d'accès dépendant du signal de détection délivré par le capteur, la partie conservable du support étant apte à être mécaniquement séparée de la partie jetable pour former une clé d'accès permettant, en fonction des informations d'accès, l'accès ou non à la zone à accès restreint. La partie conservable est ainsi configurée pour former une unité fonctionnelle, indépendante de la partie jetable. Suite à sa séparation de la partie jetable, la partie conservable forme une clé qui peut être utilisée indépendamment de la partie jetable. Par séparation, il est entendu une séparation définitive ou une déconnexion permettant de reconnecter la partie clé à une nouvelle partie capteur jetable qui permettra de réutiliser la clé ultérieurement.

En effet, un tel dispositif d'accès combine :
- un capteur destiné à mesurer, de manière autonome et embarquée, chaque paramètre de santé du sujet, par exemple mesurer la présence d'au moins un analyte prédéterminé dans un échantillon biologique prélevé sur le sujet ; et
- un étage de contrôle d'accès, destiné à stocker, des informations dépendant d'une telle détection, couplé ou non à des données biométriques.

Par séparation de la partie conservable (qui est munie de l'étage de contrôle d'accès) de la partie jetable (qui contient le biocapteur usagé), on obtient une clé d'accès susceptible d'être utilisée comme un permis d'accès.

De cette façon, l'intervention d'un biologiste n'est pas requise, et le sujet est susceptible d'obtenir rapidement et automatiquement la clé d'accès. En outre, la partie jetable, potentiellement contaminée (voire contaminante), peut simplement être jetée, par exemple dans un conteneur sécurisé fourni sur le site du test, et/ou plongée dans une solution désinfectante pour neutraliser son éventuelle dangerosité.

En fonction des informations d'accès stockées sur la clé d'accès, par exemple lues par un lecteur agencé à une entrée d'une zone à accès restreint, le sujet pourra ou non être autorisé à accéder à ladite zone.

Il en résulte que le dispositif d'accès selon l'invention présente les avantages suivants :
- ergonomie et hygiène : le test est potentiellement non invasif (par exemple lorsqu'il est basé sur l'utilisation de la salive du sujet en tant qu'échantillon biologique), permettant au sujet humain lui-même de l'effectuer. Le capteur potentiellement souillé est détaché et jeté. En outre, le filtrage des sujets testés peut se faire sans aucune intervention humaine, par exemple au moyen de portiques automatisés ;
- coût de fabrication : de par sa structure, un tel dispositif est simple à réaliser, et présente donc un faible coût de fabrication ;
- rapidité du test : le capteur peut être optimisé de façon à délivrer un signal de détection au bout de seulement quelques minutes. En outre, sur les territoires où la législation le permet, le test peut être réalisé sans l'intervention d'un biologiste, de sorte que la clé d'accès est susceptible d'être obtenue rapidement.

Suivant d'autres aspects avantageux de l'invention, le dispositif d'accès comporte une ou plusieurs des caractéristiques suivantes, prise(s) isolément ou suivant toutes les combinaisons techniquement possibles :
- le capteur est destiné à être mis en contact avec un échantillon biologique prélevé sur un sujet, le capteur étant, en outre, configuré pour délivrer un signal de détection représentatif de la présence ou non d'au moins un analyte prédéterminé dans l'échantillon biologique, le signal de détection étant également représentatif de la présence ou non d'au moins un analyte prédéterminé dans l'échantillon biologique ;
- les informations d'accès comprennent le signal de détection et/ou des données relatives à la présence ou non d'au moins un analyte prédéterminé dans un échantillon biologique prélevé sur le sujet et/ou des données relatives à un horodatage et à une durée de validité d'une autorisation d'accès et/ou des données relatives à un lieu de validité de l'autorisation d'accès et, de préférence, un identifiant unique du sujet ;
- l'étage de contrôle d'accès est, en outre, configuré pour stocker un identifiant unique du sujet sur lequel l'échantillon biologique a été prélevé ;
- l'étage de contrôle d'accès est configuré pour recevoir le signal de détection, l'étage de contrôle d'accès étant, en outre, configuré pour établir, avec une unité de traitement de données externe, une liaison sans fil, de préférence cryptée, et pour transmettre, à destination de l'unité de traitement de données, via la liaison sans fil, le signal de détection reçu en provenance du capteur, l'étage de contrôle d'accès étant également configuré pour recevoir, depuis l'unité de traitement de données, via la liaison sans fil, un signal d'analyse établi à partir du signal de détection et comprenant les informations d'accès ;
- l'étage de contrôle d'accès est également configuré pour recevoir de l'énergie via la liaison sans fil, l'étage de contrôle d'accès étant configuré pour prélever au moins une partie de l'énergie reçue afin de générer un signal d'interrogation du capteur, le capteur étant configuré pour délivrer le signal de détection suite à la réception du signal d'interrogation généré par l'étage de contrôle d'accès ;
- l'étage de contrôle d'accès est électriquement relié au capteur au moyen d'un bus de connexion, la partie conservable du support étant configurée pour être séparée de la partie jetable par cassure suivant une ligne de fracture prédéterminée, le bus de connexion étant configuré pour se sectionner, de préférence au niveau de la ligne de fracture, lors de la séparation de la partie conservable de la partie jetable.

A titre d'exemple, dans ce mode de réalisation particulier, la partie conservable peut être composée de plusieurs parties détachables contenant chacune un étage de contrôle d'accès, disposées les unes à la suite des autres et reliées entre elles par le bus de connexion de façon à recevoir des informations à partir d'un même échantillon. Il est important de noter qu'une fois le test réalisé, la fracture au niveau du bus de connexion n'affecte pas l'information déjà transmise à l'un ou aux étage(s) de contrôlé d'accès ; ou
- l'étage de contrôle d'accès est électriquement relié au capteur au moyen d'un bus de connexion, la partie conservable du support étant configurée pour être séparée de la partie jetable par décollement au niveau d'une zone de collage prédéterminée, la continuité électrique du bus de connexion dans la zone de collage étant assurée par une colle conductrice ou par des vias configurés pour rompre lors du décollement. Il est important de noter qu'une fois le test réalisé, le décollement au niveau du bus de connexion n'affecte pas l'information déjà transmise à l'étage de contrôlé d'accès ; ou
- l'étage de contrôle d'accès est électriquement relié au capteur au moyen d'un bus de connexion, la partie conservable du support étant configurée pour être séparée de la partie jetable par déconnexion, les deux parties sont reliées entre elles par un connecteur multi contact, tel qu'à titre d'exemple un système de clé USB ou une carte mémoire. La partie mâle du connecteur peut être contenue indifféremment sur la partie conservable ou sur la partie jetable. Dans ce cas, le bus de connexion est configuré pour être discontinué de préférence au niveau de la zone de déconnexion, lors de la séparation de la partie conservable 26 de la partie jetable 24 , de cette manière la partie PASS conservable, une fois réinitialisée, est réutilisable avec une nouvelle partie capteur jetable. Il est important de noter qu'une fois le test réalisé, la déconnexion n'affecte pas l'information déjà transmise à l'étage de contrôlé d'accès ;
- l'étage de contrôle d'accès forme au moins une partie d'une radio-étiquette, de préférence apte à mettre en œuvre une communication en champ proche ;
- le capteur et la partie conservable du support sont à l'écart l'un de l'autre, une distance entre le capteur et la partie conservable du support étant supérieure à une limite de contamination prédéterminée.

En outre, l'invention a pour objet un système de génération de clé d'accès comprenant le dispositif d'accès tel que défini ci-dessus, et une unité de traitement de données configurée pour recevoir le signal de détection, pour générer, à partir du signal de détection reçu, un signal d'analyse comprenant les informations d'accès, et pour fournir le signal d'analyse à l'étage de contrôle d'accès.

En outre, l'invention a pour objet un procédé de contrôle d'accès pour autoriser ou non l'accès d'un sujet à une zone à accès restreint, le procédé mettant en œuvre le dispositif d'accès tel que défini ci-dessus et comprenant les étapes :
- mesure, au moyen du capteur d'au moins un paramètre de santé du sujet ;
- génération des informations d'accès en fonction du signal de détection ;
- stockage des informations d'accès dans l'étage de contrôle d'accès ; et
- séparation de la partie conservable du support de la partie jetable pour former la clé d'accès.

Suivant un autre aspect avantageux de l'invention, le procédé de contrôle d'accès comporte une ou plusieurs des caractéristiques suivantes, prise(s) isolément ou suivant toutes les combinaisons techniquement possibles :
- le procédé comprend, en outre, un enregistrement, dans l'étage de contrôle d'accès, d'un identifiant unique du sujet sur lequel l'échantillon biologique a été prélevé ;
- le procédé comprend, en outre, les étapes :
   - transmission du signal de détection délivré par le capteur à destination d'une unité de traitement de données, via une liaison sans fil, de préférence cryptée ;
   - analyse, par l'unité de traitement de données, du signal de détection délivré par le capteur, pour déterminer une valeur de chaque paramètre de santé, et génération des informations d'accès en fonction de l'analyse du signal de détection ; et
   - après la génération des informations d'accès par l'unité de traitement de données, transmission à destination de l'étage de contrôle d'accès d'un signal d'analyse comprenant les informations d'accès générées, depuis l'unité de traitement de données, via la liaison sans fil ;
- le procédé comprend, en outre, les étapes :
   - présentation de la clé d'accès formée à un lecteur équipant une entrée de la zone à accès restreint ;
   - lecture, au moyen du lecteur, des informations d'accès stockées dans l'étage de contrôle d'accès de la clé d'accès ;
   - autorisation ou non de l'accès du sujet à la zone à accès restreint en fonction des informations d'accès lues ;
- durant l'autorisation ou non de l'accès à la zone à accès restreint, l'accès à la zone à accès restreint est autorisé si les informations d'accès indiquent l'absence d'au moins un analyte prédéterminé dans un échantillon biologique prélevé sur le sujet ou si une valeur d'un paramètre de santé appartient à un intervalle ou un seuil prédéterminé, et est interdit sinon.

### BRÈVE DESCRIPTION DES DESSINS

L'invention sera mieux comprise à l'aide de la description qui va suivre, donnée uniquement à titre d'exemple non limitatif et faite en se référant aux dessins annexés sur lesquels :
- la figure 1 est une représentation schématique d'un système de génération de clé d'accès selon l'invention ;
- la figure 2 est une vue schématique en perspective d'une ligne de fracture d'un support d'un premier mode de réalisation d'un dispositif d'accès du système de génération de clé d'accès de la figure 1 ;
- la figure 3 est une vue schématique en perspective d'une zone de décollement d'un support d'un deuxième mode de réalisation d'un dispositif d'accès du système de génération de clé d'accès de la figure 1 ; et
- la figure 4 montre la partie conservable du support qui est configurée pour être séparée de la partie jetable par déconnexion.

### DESCRIPTION DÉTAILLÉE

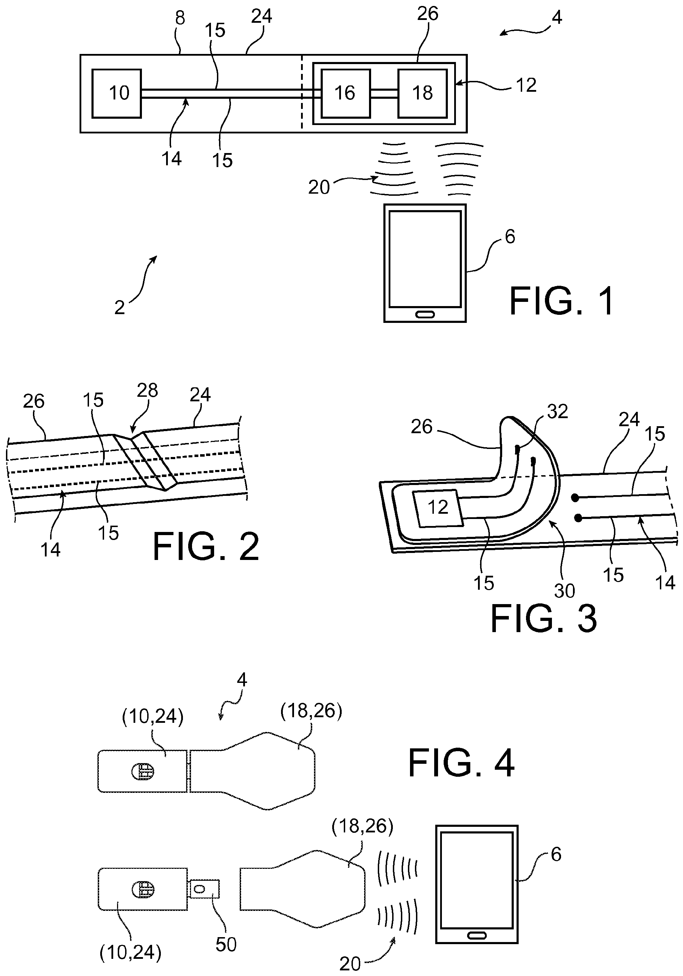

Un système 2 de génération de clé d'accès selon l'invention est illustré par la figure 1.

Un tel système de génération 2 est, par exemple, destiné à être utilisé en tant qu'autotest rapide immunologique de charge virale par bio-détection électronique d'un échantillon de salive.

Le système de génération 2 est apte à fournir une clé d'accès permettant de déterminer si un sujet est autorisé ou non à accéder à une zone à accès restreint. Comme cela ressortira de la description qui va suivre, une telle clé d'accès est un objet physique, et non pas virtuel.

Le système de génération 2 comprend un dispositif d'accès 4 et une unité de traitement de données 6 configurée pour coopérer avec le dispositif d'accès 4 pour paramétrer la clé d'accès.

### Structure

### Dispositif d'accès

Comme cela ressortira de la description qui va suivre, le dispositif d'accès 6 est un dispositif portable et mono-usage.

Plus précisément, par « mono-usage », il est entendu au sens de la présente invention que, pour un dispositif d'accès 4 donné, un seul sujet, ou encore un seul échantillon biologique, est testé, un capteur 10 du dispositif d'accès étant, de préférence, jeté (voire détruit) après un tel test. En revanche, et comme cela sera décrit ultérieurement, la clé d'accès en elle-même est susceptible d'être présentée à de nombreuses reprises lors de contrôles destinés à déterminer si le sujet est autorisé ou non à accéder à une ou des zone(s) à accès restreint, dans une fenêtre temporelle restreinte ou non.

Le dispositif d'accès 4 comprend un support 8, un capteur 10 et un circuit intégré 12 électriquement relié au capteur 10 au moyen d'un bus de connexion 14.

Le capteur 10 est configuré pour mesurer au moins un paramètre de santé d'un sujet, chaque paramètre de santé étant représentatif d'un état de santé du sujet.

Par exemple, le capteur 10 est configuré pour mesurer une température ou une tension artérielle du sujet (avec ou sans contact avec le sujet), ou encore un pH d'un échantillon biologique issu du sujet.

Le capteur 10 est sensible à au moins un analyte prédéterminé. Le capteur 10 est destiné à être mis en contact avec un échantillon biologique à tester, prélevé sur le sujet, pour mesurer la présence ou non de chaque analyte prédéterminé, voire sa concentration dans l'échantillon biologique.

Par exemple, l'analyte prédéterminé est un composé tel qu'un acide nucléique ou une protéine représentatif d'un contaminant microbiologique, tel que qu'un contaminant viral ou bactérien, et/ou un anticorps spécifique à un antigène donné, et/ou un analyte représentatif d'un état de santé altéré et/ou d'une intoxication et/ou d'une concentration ou d'une valeur d'un paramètre physicochimique.

En outre, le capteur 10 est configuré pour délivrer un signal de détection représentatif de la mesure de chaque paramètre de santé prédéterminé. Par exemple, le capteur 10 est configuré de sorte que, pour chaque analyte prédéterminé, le signal de détection soit représentatif de la présence et/ou de la concentration de l'analyte dans l'échantillon biologique.

A titre d'exemple, l'échantillon biologique est un fluide corporel, tel que l'haleine, la salive, les expectorations, le sébum, la sueur, le sang ou les exsudats d'une plaie. Par exemple, l'échantillon biologique est issu d'un prélèvement des voies rhinopharyngées ou de la sphère buccale du sujet (salive, expectorations, etc.).

Par exemple, dans le cas où le capteur 10 est sensible à au moins un analyte prédéterminé, ledit capteur 10 comprend une surface sensible comportant un feuillet de graphène déposé sur substrat rigide (par exemple, du silicium) ou sur substrat souple (polymère). Le graphène est un matériau particulièrement avantageux pour l'invention de par sa production de masse permettant le développement d'un capteur précis pour un usage unique avec un coût réduit, et son utilisation dans le domaine de la chimie de surface. Dans ce cas, le feuillet de graphène est recouvert d'une couche mince présentant des groupes fonctionnels (par exemple un brin d'ADN, un oligonucléotide, un aptamère, un peptide, un enzyme ou un anticorps) spécifiques d'un analyte donné. Si l'échantillon biologique comprend l'analyte, une réaction de reconnaissance biologique entre le groupe fonctionnel et ledit analyte se produit, conduisant à une immobilisation de l'analyte qui se traduit par une variation de la charge électrostatique induite en surface du feuillet de graphène. En particulier, une telle variation de charge électrostatique est d'autant plus importante que la concentration en analyte dans l'échantillon biologique est importante. Cette variation de charge électrostatique conduit à un changement de conductance électrique du feuillet de graphène, dont l'amplitude traduit la concentration en analyte. Un tel changement de conductance est susceptible d'être déterminé à partir du signal de détection délivré par le capteur 10.

Le circuit intégré 12 est configuré pour recevoir le signal de détection délivré par le capteur 10 et acheminé par le bus de connexion 14, ce dernier étant par exemple réalisé au moyen de pistes 15 électriquement conductrices.

Le circuit intégré 12 est également configuré pour échanger, par communication sans fil, des signaux avec l'unité de traitement de données 6 pour la génération de la clé d'accès.

Avantageusement, le module de communication sans fil 12 est également configuré pour recevoir, sans fil, de l'énergie depuis l'unité de traitement de données 6. Une telle transmission d'énergie est, par exemple, réalisée par induction.

La mise en œuvre d'une telle transmission d'énergie sans fil est avantageuse, dans la mesure où l'implémentation de moyens de stockage d'énergie électrique au niveau du dispositif d'accès 4 n'est plus nécessaire.

A titre d'exemple, le circuit intégré 12 est configuré pour prélever de l'énergie sur une liaison sans fil 20 décrite ultérieurement.

Le circuit intégré 12 comprend un étage de numérisation 16 et un étage de contrôle d'accès 18 électriquement reliés entre eux.

L'étage de numérisation 16 est connecté au capteur 10 pour recevoir le signal de détection analogique délivré par le capteur 10, et pour le convertir en un signal de détection numérique appliqué en entrée de l'étage de contrôle d'accès 18.

En variante, l'étage de numérisation 16 n'est pas intégré au circuit intégré 12, mais est mis en œuvre au moyen d'un composant électronique indépendant. Selon une autre variante, la fonction de numérisation est réalisée par l'étage de contrôle d'accès 18 lui-même.

L'étage de contrôle d'accès 18 est configuré pour générer un signal d'interrogation du capteur 10. Dans ce cas, le capteur 10 est configuré pour délivrer le signal de détection suite à la réception du signal d'interrogation délivré par l'étage de contrôle d'accès 18. En particulier, l'étage de contrôle d'accès 18 est configuré pour générer le signal d'interrogation à partir d'au moins une partie de l'énergie reçue par le circuit intégré 12 depuis l'unité de traitement de données 6.

En outre, l'étage de contrôle d'accès 18 est configuré pour établir, avec l'unité de traitement de données 6, une liaison sans fil 20, de préférence cryptée. En outre, l'étage de contrôle d'accès 18 est configuré pour transmettre, à destination de l'unité de traitement de données 6, le signal de détection reçu en provenance du capteur 10, via la liaison sans fil 20.

Une telle liaison sans fil 20 est, notamment, établie au moyen d'une antenne (non représentée) de l'étage de contrôle d'accès 18.

Le cryptage de la liaison sans fil 20 est avantageux, dans la mesure où une telle liaison voit transiter un signal de détection dont peuvent être extraites des données sensibles, par exemple confidentielles, relatives au sujet sur lequel a été prélevé l'échantillon biologique.

De préférence, l'étage de contrôle d'accès 18 est également configuré pour transmettre, à destination de l'unité de traitement de données 6, via la liaison sans fil 20, une information relative au capteur 10. Une telle information relative au capteur 10 est, par exemple, enregistrée dans l'étage de contrôle d'accès 18 au cours de la fabrication du dispositif d'accès 4, et est susceptible d'être utilisée par l'unité de traitement de données 6 lors de l'analyse du signal de détection, comme cela sera décrit ultérieurement.

L'étage de contrôle d'accès 18 est également configuré pour recevoir, depuis l'unité de traitement de données, via la liaison sans fil 20, un signal d'analyse établi à partir du signal de détection, et comprenant les informations d'accès.

Avantageusement, l'étage de contrôle d'accès 18 est, en outre, configuré pour stocker un identifiant unique du sujet dont le(s) paramètre(s) de santé est (sont) mesuré(s). Par exemple, l'identifiant unique est transmis à l'étage de contrôle d'accès 18 par l'unité de traitement de données 6, via la liaison sans fil 20.

Avantageusement, l'étage de contrôle d'accès 18 forme au moins une partie d'une radio-étiquette (également appelée « tag RFID », l'acronyme étant issu de l'expression anglaise *Radio Frequency IDentification,* signifiant radio-identification). Dans ce cas, la radio-étiquette est, de préférence, apte à mettre en œuvre une communication en champ proche (également appelée « NFC », acronyme de l'expression anglaise *Near Field Communication).* En variante, la radio-étiquette est apte à mettre en œuvre une communication sans fil présentant une fréquence de porteuse appartenant au domaine ultra-haute fréquence, en particulier comprise 860 MHz (mégahertz) et 960 MHz, par exemple une communication sans fil telle que définie par des standards EPCglobal ou ISO 18000-6c.

Le recours à la technologie RFID est particulièrement avantageux, notamment du point de vue des coûts de fabrication. En effet, une telle caractéristique confère une grande simplicité de fabrication au dispositif d'accès 4 (notamment au module de communication sans fil 12). Il en résulte que le dispositif d'accès 4 selon l'invention est susceptible d'être produit de façon massive et à très faible coût, les radioétiquettes étant généralement très peu onéreuses.

En outre, le recours à la technologie RFID octroie également la capacité de recourir à un cryptage des données stockées et des signaux échangés par voie hertzienne. De cette façon, le(s) résultat(s) du test de l'échantillon biologique sont potentiellement inaltérable(s) et confidentiel(s).

Le support 8 présente, de préférence, une forme allongée. Par exemple, le support 8 se présente sous la forme d'une bandelette, d'un bâtonnet, d'un écouvillon, d'une languette, etc.

Le support 8 comprend une partie jetable 24 et une partie conservable 26 mécaniquement reliées entre elles.

Plus précisément, et comme cela apparaît sur la figure 1, la partie jetable 24 est équipée du capteur 10. En outre, la partie conservable 26 est équipée de l'étage de contrôle d'accès 18. Par ailleurs, l'étage de numérisation 16, lorsqu'il n'est pas inclus dans le circuit intégré 12, est porté soit par la partie jetable 24, soit par la partie conservable 26 du support 8.

La partie conservable 26 du support 8 est apte à être mécaniquement séparée de la partie jetable 24 pour former une clé d'accès permettant, en fonction des informations d'accès, l'accès ou non à la zone à accès restreint.

Bien évidemment, la clé d'accès en elle-même n'est pas à usage unique, et est susceptible d'être présentée lors d'une pluralité de contrôles chacun destiné à autoriser ou non l'accès du sujet associé à ladite clé d'accès à une même zone à accès restreint ou à des zones à accès restreint distinctes.

Plus précisément, la partie conservable 26 du support 8 est configurée pour être mécaniquement séparée de la partie jetable 24 par cassure, découpage, déchirure ou décollement. En outre, le bus de connexion 14 est configuré pour se sectionner lors d'une telle séparation, de façon, notamment, à permettre une séparation facile de la partie jetable 24 et de la partie conservable 26.

Selon un exemple illustré par la figure 2, la partie conservable 26 du support 8 est configurée pour être séparée de la partie jetable 24 par cassure, plus précisément par cassure suivant une ligne de fracture 28 prédéterminée. Dans ce cas, le bus de connexion 14 est configuré pour se sectionner, en particulier au niveau de la ou des ligne(s) de fracture 28.

Dans ce cas, le support 8 est, par exemple, une pièce en plastique polymère moulée dont la structure présente une encoche définissant la ligne de fracture 28 pour permettre la cassure lors d'une torsion mécanique appliquée sur le support 8. Les pistes 15 sont, par exemple, réalisés dans un matériau conducteur facilement sécable, par exemple un polymère conducteur incorporant des particules d'argent dans une goulotte plastique enterrée dans le support 8. De cette façon, la clé d'accès est susceptible d'être utilisée à la manière d'un badge d'accès ou d'une carte de crédit prédécoupée.

Selon un autre exemple, illustré par la figure 3, la partie conservable 26 du support 8 est configurée pour être séparée de la partie jetable 24 par décollement au niveau d'une zone de collage 30 prédéterminée. Dans ce cas, lorsque la partie jetable 24 et la partie conservable 26 du support 8 ne sont pas mécaniquement séparées, la continuité électrique du bus de connexion 14 au niveau de la zone de collage 30 est assurée par une colle conductrice (par exemple une colle conductrice chargée aux microparticules d'argent), et/ou des vias 32. De tels vias 32 sont configurés pour rompre lors du décollement de la partie conservable 26. De la même façon, la colle conductrice est configurée de façon à ne plus assurer la continuité électrique du bus de connexion 14 à l'issue du décollement de la partie conservable 26.

Dans ce cas, la partie conservable 26 est, par exemple, un support papier ou polymère comportant l'antenne de l'étage de contrôle d'accès sous la forme d'une structure imprimée souple réalisée en encre conductrice ou en couche mince métallique gravée, communément désignée par l'expression anglaise « *inlay RFID* ». Avantageusement, pour favoriser le décollement de la partie conservable 26, la partie jetable 24 est munie, au niveau de la zone de collage 30, d'une couche antiadhésive adéquate.

Avantageusement, la clé d'accès présente une surface adhésive. De cette façon, la clé d'accès est susceptible d'être utilisée à la manière d'un autocollant et, par exemple, collée sur une pièce d'identité, une carte d'embarquement, etc.

Selon un exemple illustré par la figure 4, la partie conservable 26 du support 8 est configurée pour être séparée de la partie jetable 24 par déconnexion, plus précisément les deux parties sont reliées entre elles par un connecteur multi contact 50, tel qu'à titre d'exemple un système de clé USB ou une carte mémoire. La partie mâle du connecteur peut être contenue indifféremment sur la partie conservable 26 ou sur la partie jetable 24. Dans ce cas, le bus de connexion est configuré pour être discontinué de préférence au niveau de la zone de déconnexion, lors de la séparation de la partie conservable 26 de la partie jetable 24. La partie conservable 26, ainsi déconnectée de la partie jetable 24, peut alors être utilisée pour coopérer avec l'unité de traitement 6.

Selon tous les exemples de réalisation, la partie conservable 26 est configurée pour exercer la fonction de clé indépendamment de la partie jetable. Les éléments fonctionnels mentionnés ci-dessus (par exemple circuit intégré, stockage d'énergie, stockage d'information) sont ainsi répartis entre la partie jetable et la partie conservable de façon à permettre à la partie conservable d'exercer sa fonction de clé indépendamment.

De préférence, le dispositif d'accès 4 est tel que, avant la séparation de la partie jetable 24 de la partie conservable 26 du support 8, le capteur 10 et la partie conservable 26 du support 8 sont à l'écart l'un de l'autre, la distance entre le capteur 10 et la partie conservable 26 du support 8 étant supérieure à une limite de contamination prédéterminée. Ceci est avantageux, dans la mesure où une contamination de la partie conservable 26, durant l'utilisation du dispositif d'accès 4 pour tester l'échantillon biologique, est ainsi potentiellement évitée.

### Unité de traitement de données

Comme indiqué précédemment, l'unité de traitement de données 6, représentée sur la figure 1, est configurée pour recevoir le signal de détection depuis l'étage de contrôle d'accès 18 via la liaison sans fil 20, et pour analyser ledit signal de détection. En particulier, l'unité de traitement de données 6 est apte à analyser le signal de détection, de préférence en correspondance avec le capteur 10 dont est issu ledit signal de détection, pour déterminer les paramètres de santé, par exemple déterminer la présence et/ou la concentration de chaque analyte prédéterminé correspondant dans l'échantillon biologique.

Par exemple, l'unité de traitement de données 6 est un smartphone exécutant une application dédiée de traitement de signaux de détection délivrés par des capteurs 10 prédéterminés.

L'unité de traitement de données 6 est également configurée pour générer, à partir d'une telle analyse du signal de détection reçu, des informations d'accès correspondantes.

De préférence, les informations d'accès comprennent des données relatives à la présence ou non de chaque analyte prédéterminé dans l'échantillon biologique, voire à sa concentration dans l'échantillon biologique, ou encore relatives à la valeur de paramètres physiques, physiologiques ou biologiques prédéterminés.

De préférence encore, les informations d'accès comprennent des données relatives à un horodatage et à une durée de validité d'une autorisation d'accès et/ou des données relatives à un lieu de validité de l'autorisation d'accès. Ceci est avantageux, dans la mesure où la validité des résultats du test du sujet et/ou de l'échantillon biologique prélevé sur ledit sujet, effectué au moyen du système de génération 2, se trouve ainsi limitée à une fenêtre spatio-temporelle donnée. Une telle fenêtre spatio-temporelle est, par exemple, associée à un évènement donné, tel que, de façon non limitative, une phase d'embarquement d'un vol commercial donné. Bien évidemment, une telle fenêtre spatio-temporelle est susceptible d'être associée à tout évènement nécessitant un contrôle de sujets pour leur octroyer la possibilité d'accéder à une zone à accès restreint.

En outre, l'unité de traitement de données 6 est configurée pour transmettre un signal d'analyse à l'étage de contrôle d'accès 18, via la liaison sans fil 20, le signal d'analyse comprenant les informations d'accès générées. Il en résulte que le signal d'analyse dépend du signal de détection délivré par le capteur 10.

Avantageusement, les informations d'accès comprennent également l'identifiant unique du sujet dont les paramètres de santé ont été mesurés (par exemple à partir d'un échantillon biologique prélevé sur ledit sujet). Une telle caractéristique est avantageuse, dans la mesure où elle renforce la traçabilité de la clé d'accès, et réduit les risques de fraude.

Par exemple, dans ce dernier cas, l'unité de traitement de données 6 est configurée pour offrir la possibilité au sujet de s'identifier au moyen d'un portail sécurisé. Bien entendu, tout autre moyen d'identifier le sujet de façon univoque est envisageable.

### Fonctionnement

Le fonctionnement du système de génération 2 va maintenant être décrit.

Pour déterminer si un sujet est autorisé ou non à accéder à une zone à accès restreint, chaque paramètre de santé prédéterminé du sujet est mesuré au moyen du capteur 10 du dispositif d'accès 4 de ce système de génération 2. Par exemple, un échantillon biologique est prélevé sur ledit sujet, puis le capteur 10 est mis en contact avec l'échantillon biologique prélevé.

Puis, le capteur 10 délivre le signal de détection correspondant, qui est numérisé par l'étage de numérisation 16 et appliqué en entrée de l'étage de contrôle d'accès 18. Puis, le signal de détection délivré par le capteur 10 est analysé pour déterminer chaque paramètre de santé, par exemple pour déterminer la présence ou non d'au moins un analyte dans l'échantillon biologique, et pour générer des informations d'accès dépendant d'un résultat de ladite analyse du signal de détection.

En particulier, une liaison sans fil 20, de préférence cryptée, est établie entre l'étage de contrôle d'accès 18 et l'unité de traitement de données 6. Dans ce cas, le signal de détection reçu par l'étage de contrôle d'accès 18 en provenance du capteur 10 est transmis à destination de l'unité de traitement de données 6 via la liaison sans fil 20, de sorte que l'analyse du signal de détection et la génération des informations d'accès sont mises en œuvre par l'unité de traitement de données 6.

Puis, les informations d'accès sont stockées dans l'étage de contrôle d'accès 18. De préférence, les informations d'accès sont stockées dans l'étage de contrôle d'accès 18 sous forme cryptée. De cette façon, les informations d'accès stockées dans l'étage de contrôle d'accès 18 ne peuvent être lues que par des lecteurs configurés spécifiquement pour les décrypter. Ceci augmente la sécurité de la clé d'accès, et réduit les chances de réécriture des informations qu'elle contient par un tiers malveillant.

En particulier, pour permettre un tel stockage des informations d'accès, l'unité de traitement de données 6 envoie, via la liaison sans fil 20, à destination de l'étage de contrôle d'accès 18, un signal d'analyse comprenant les informations d'accès.

Enfin, la partie conservable 26 du support 4 est séparée de la partie jetable 24 pour former la clé d'accès.

De préférence, le signal d'analyse comprend, en outre, un identifiant unique du sujet sur lequel l'échantillon biologique a été prélevé. Dans ce cas, l'unité de traitement de données 6 transmet également l'identifiant unique pour son stockage dans l'étage de contrôle d'accès.

Une fois la clé d'accès détachée de la partie jetable 24 du support 8, la clé d'accès est présentée à un lecteur équipant une entrée de la zone à accès restreint.

Puis, le lecteur lit les informations d'accès stockées dans l'étage de contrôle d'accès 18 de la clé d'accès, à la suite de quoi, en fonction des informations d'accès lues, le sujet est autorisé ou non à accéder à la zone à accès restreint.

Par exemple, l'accès à la zone à accès restreint est autorisé si les informations d'accès indiquent l'absence d'au moins un analyte dans l'échantillon biologique, ou encore si une valeur d'un paramètre de santé appartient à un intervalle ou un seuil prédéterminé, et est interdit sinon.

Par ailleurs, la partie jetable 24 du support 8 qui a été séparée de la partie conservable 26 du support 8 est, de préférence, acheminée vers une filière de traitement des déchets biologiques, et/ou plongée dans une solution désinfectante pour neutraliser son éventuelle dangerosité.

En variante, l'unité de traitement de données est intégrée dans le lecteur destiné à autoriser ou non l'accès du sujet à la zone à accès restreint, le lecteur réalisant alors l'analyse du signal de détection.

Dans ce cas, le signal de détection stocké dans l'étage de contrôle d'accès 18 forme au moins une partie des informations d'accès. Dans une telle variante, la liaison sans fil est directement établie avec le lecteur pour la transmission du signal de détection.

En outre, dans ce cas, le dispositif d'accès 4 est, de préférence, équipé d'un organe de stockage d'énergie destiné à fournir à l'étage de contrôle d'accès l'énergie nécessaire à son fonctionnement.

Un tel organe de stockage d'énergie est, par exemple, chargé au moment de la fabrication du dispositif d'accès 4. Selon un autre exemple, l'organe de stockage d'énergie est chargé au moyen d'une liaison sans fil, ladite liaison sans fil étant par exemple établie pour transmettre, à destination de l'étage de contrôle d'accès 18, l'identifiant unique du sujet, avant la mesure des paramètres de santé dudit sujet par le capteur 10.

Selon une autre variante, le système 2 de génération de clé d'accès est dépourvu d'unité de traitement de données, l'étage de contrôle d'accès 18 étant configuré pour analyser le signal de détection afin d'établir les informations d'accès.

Dans ce cas également, un organe de stockage d'énergie est avantageusement prévu dans le dispositif d'accès 4 pour fournir à l'étage de contrôle d'accès 18 l'énergie nécessaire à son fonctionnement.

## Revendications

1. Dispositif d'accès (4) portable à une zone à accès restreint, le dispositif d'accès est mono-usage et comprend un support (8), un capteur (10) et un étage de contrôle d'accès (18),
le dispositif d'accès portable est mono-usage en ce que le capteur (10) est destiné à être mis en contact avec un échantillon biologique prélevé sur un sujet,
le support (8) comprenant une partie jetable (24) et une partie conservable (26),
le capteur (10) étant porté par la partie jetable (24) du support (8) et étant configuré pour mesurer au moins un paramètre de santé d'un sujet, et pour délivrer un signal de détection représentatif de la mesure de chaque paramètre de santé,
l'étage de contrôle d'accès (18) étant porté par la partie conservable (26) du support (8) et étant configuré pour stocker, de préférence sous forme cryptée, des informations d'accès dépendant du signal de détection délivré par le capteur (10),
la partie conservable (26) du support (8) étant apte à être mécaniquement séparée de la partie jetable (24) pour former une clé d'accès permettant, en fonction des informations d'accès, l'accès ou non à la zone à accès restreint.

2. Dispositif d'accès (4) selon la revendication 1, dans lequel le capteur (10) est configuré pour délivrer un signal de détection représentatif de la présence ou non d'au moins un analyte prédéterminé dans l'échantillon biologique, le signal de détection étant également représentatif de la présence ou non d'au moins un analyte prédéterminé dans l'échantillon biologique.

3. Dispositif d'accès (4) selon la revendication 1 ou 2, dans lequel le capteur (10) est composé d'un feuillet de graphène, ledit feuillet de graphène étant recouvert d'une couche mince présentant des groupes fonctionnels spécifiques d'un analyte donné.

4. Dispositif d'accès (4) selon la revendication 1, 2 ou 3, dans lequel les informations d'accès comprennent le signal de détection et/ou des données relatives à la présence ou non d'au moins un analyte prédéterminé dans un échantillon biologique prélevé sur le sujet et/ou des données relatives à un horodatage et à une durée de validité d'une autorisation d'accès et/ou des données relatives à un lieu de validité de l'autorisation d'accès et, de préférence, un identifiant unique du sujet.

5. Dispositif d'accès (4) selon l'une quelconque des revendications 1 à 4, dans lequel l'étage de contrôle d'accès (18) est, en outre, configuré pour stocker un identifiant unique du sujet.

6. Dispositif d'accès (4) selon l'une quelconque des revendications 1 à 5, dans lequel l'étage de contrôle d'accès (18) est configuré pour recevoir le signal de détection,
l'étage de contrôle d'accès (18) étant, en outre, configuré pour établir, avec une unité de traitement de données (6) externe, une liaison sans fil (20), de préférence cryptée, et pour transmettre, à destination de l'unité de traitement de données (6), via la liaison sans fil (20), le signal de détection reçu en provenance du capteur (10),
l'étage de contrôle d'accès (18) étant également configuré pour recevoir, depuis l'unité de traitement de données (6), via la liaison sans fil (20), un signal d'analyse établi à partir du signal de détection et comprenant les informations d'accès.

7. Dispositif d'accès (4) selon la revendication 6, dans lequel l'étage de contrôle d'accès (18) est également configuré pour recevoir de l'énergie via la liaison sans fil (20), l'étage de contrôle d'accès étant configuré pour prélever au moins une partie de l'énergie reçue afin de générer un signal d'interrogation du capteur (10), le capteur (10) étant configuré pour délivrer le signal de détection suite à la réception du signal d'interrogation généré par l'étage de contrôle d'accès (18).

8. Dispositif d'accès (4) selon l'une quelconque des revendications 1 à 7, dans lequel l'étage de contrôle d'accès (18) est électriquement relié au capteur (10) au moyen d'un bus de connexion (14), la partie conservable (26) du support (8) étant configurée pour être séparée de la partie jetable (24) par cassure suivant une ligne de fracture (28) prédéterminée, le bus de connexion (14) étant configuré pour se sectionner, de préférence au niveau de la ligne de fracture (28), lors de la séparation de la partie conservable (26) de la partie jetable (24).

9. Dispositif d'accès (4) selon l'une quelconque des revendications 1 à 7, dans lequel l'étage de contrôle d'accès (18) est électriquement relié au capteur (10) au moyen d'un bus de connexion (14), la partie conservable (26) du support (8) étant configurée pour être séparée de la partie jetable (24) par décollement au niveau d'une zone de collage (30) prédéterminée, la continuité électrique du bus de connexion (14) dans la zone de collage (30) étant assurée par une colle conductrice ou par des vias configurés pour rompre lors du décollement.

10. Dispositif d'accès (4) selon l'une quelconque des revendications 1 à 7, dans lequel l'étage de contrôle d'accès (18) est électriquement relié au capteur (10) au moyen d'un bus de connexion (14), la partie conservable (26) du support (8) est reliée à la partie jetable (24) par un connecteur multi contact (50), tel qu'à titre d'exemple un système de clé USB ou une carte mémoire , et la partie conservable (26) est configurée pour être séparée de la partie jetable (24) par déconnexion, le bus de connexion (14) étant configuré pour être discontinué, de préférence au niveau de la zone de déconnexion, lors de la séparation de la partie conservable (26) de la partie jetable (24) une partie mâle du connecteur (50) pouvant être contenue indifféremment sur la partie conservable (26) ou sur la partie jetable (24).

11. Dispositif d'accès (4) selon l'une quelconque des revendications 1 à 10, dans lequel l'étage de contrôle d'accès (18) forme au moins une partie d'une radio-étiquette, de préférence apte à mettre en œuvre une communication en champ proche.

12. Dispositif d'accès (4) selon l'une quelconque des revendications 1 à 11, dans lequel le capteur (10) et la partie conservable (26) du support (8) sont à l'écart l'un de l'autre, une distance entre le capteur (10) et la partie conservable (26) du support (8) étant supérieure à une limite de contamination prédéterminée.

13. Système (2) de génération de clé d'accès comprenant le dispositif d'accès (4) selon l'une quelconque des revendications 1 à 12, et une unité de traitement de données (6) configurée pour recevoir le signal de détection, pour générer, à partir du signal de détection reçu, un signal d'analyse comprenant les informations d'accès, et pour fournir le signal d'analyse à l'étage de contrôle d'accès (18).

14. Procédé de contrôle d'accès pour autoriser ou non l'accès d'un sujet à une zone à accès restreint, le procédé mettant en œuvre le dispositif d'accès (4) selon l'une quelconque des revendications 1 à 13 et comprenant les étapes :
- mesure, au moyen du capteur (10) d'au moins un paramètre de santé du sujet ;
- génération des informations d'accès en fonction du signal de détection ;
- stockage des informations d'accès dans l'étage de contrôle d'accès (18) ; et
- séparation de la partie conservable (26) du support (8) de la partie jetable (24) pour former la clé d'accès, de préférence comprenant, en outre, un enregistrement, dans l'étage de contrôle d'accès (18), d'un identifiant unique du sujet.

15. Procédé de contrôle d'accès selon la revendication 14, comprenant, en outre :
- transmission du signal de détection délivré par le capteur (10) à destination d'une unité de traitement de données (6), via une liaison sans fil (20), de préférence cryptée ;
- analyse, par l'unité de traitement de données (6), du signal de détection délivré par le capteur (10), pour déterminer une valeur de chaque paramètre de santé, et génération des informations d'accès en fonction de l'analyse du signal de détection ; et
- après la génération des informations d'accès par l'unité de traitement de données (6), transmission à destination de l'étage de contrôle d'accès (18) d'un signal d'analyse comprenant les informations d'accès générées, depuis l'unité de traitement de données (6), via la liaison sans fil.

16. Procédé de contrôle d'accès selon la revendication 14 ou 15, comprenant, en outre, les étapes :
- présentation de la clé d'accès formée à un lecteur équipant une entrée de la zone à accès restreint ;
- lecture, au moyen du lecteur, des informations d'accès stockées dans l'étage de contrôle d'accès (18) de la clé d'accès ;
- autorisation ou non de l'accès du sujet à la zone à accès restreint en fonction des informations d'accès lues, de préférence dans lequel, durant l'autorisation ou non de l'accès à la zone à accès restreint, l'accès à la zone à accès restreint est autorisé si les informations d'accès indiquent l'absence d'au moins un analyte prédéterminé dans un échantillon biologique prélevé sur le sujet ou si une valeur d'un paramètre de santé appartient à un intervalle prédéterminé, et est interdit sinon.

## Patentansprüche

1. Tragbare Zugangsvorrichtung (4) für einen Zugangsbeschränkungsbereich, wobei die Zugangsvorrichtung für den einmaligen Gebrauch bestimmt ist und eine Halterung (8), einen Sensor (10) und eine Zugangssteuerstufe (18) umfasst,
die tragbare Zugangsvorrichtung für den einmaligen Gebrauch bestimmt ist, da der Sensor (10) dazu bestimmt ist, mit einer von einem Probanden entnommenen biologischen Probe in Kontakt zu kommen,
wobei die Halterung (8) einen Einwegteil (24) und einen Aufbewahrungsteil (26) umfasst,
wobei der Sensor (10) von dem Einwegteil (24) der Halterung (8) getragen wird und so eingerichtet ist, dass er mindestens einen Gesundheitsparameter eines Probanden misst und ein Erkennungssignal liefert, das für die Messung jedes Gesundheitsparameters repräsentativ ist,
wobei die Zugangssteuerstufe (18) von dem Aufbewahrungsteil (26) der Halterung (8) getragen wird und so eingerichtet ist, dass sie Zugangsinformationen speichert, vorzugsweise in verschlüsselter Form, die von dem vom Sensor (10) gelieferten Erkennungssignal abhängig sind,
wobei der Aufbewahrungsteil (26) der Halterung (8) mechanisch von dem Einwegteil (24) getrennt werden kann, um einen Zugangsschlüssel zu bilden, der in Abhängigkeit von den Zugangsinformationen den Zugang zu dem Zugangsbeschränkungsbereich ermöglicht oder nicht.

2. Zugangsvorrichtung (4) nach Anspruch 1, wobei der Sensor (10) so eingerichtet ist, dass er ein Erkennungssignal liefert, das für das Vorhandensein oder Nichtvorhandensein von mindestens einem vorbestimmten Analyten in der biologischen Probe repräsentativ ist, wobei das Erkennungssignal auch für das Vorhandensein oder Nichtvorhandensein von mindestens einem vorbestimmten Analyten in der biologischen Probe repräsentativ ist.

3. Zugangsvorrichtung (4) nach Anspruch 1 oder 2, wobei der Sensor (10) aus einer Graphenfolie besteht, wobei die Graphenfolie mit einer dünnen Schicht bedeckt ist, die spezifische Funktionsgruppen eines bestimmten Analyten besitzt.

4. Zugangsvorrichtung (4) nach Anspruch 1, 2 oder 3, wobei die Zugangsinformationen das Erkennungssignal und/oder Daten über das Vorhandensein oder Nichtvorhandensein mindestens eines vorbestimmten Analyten in einer von dem Probanden entnommenen biologischen Probe und/oder Daten über einen Zeitstempel und eine Gültigkeitsdauer einer Zugangsberechtigung und/oder Daten über einen Gültigkeitsort der Zugangsberechtigung und vorzugsweise eine eindeutige Identifikationsnummer des Probanden umfassen.

5. Zugangsvorrichtung (4) nach einem der Ansprüche 1 bis 4, wobei die Zugangssteuerstufe (18) ferner so eingerichtet ist, dass sie eine eindeutige Kennung des Probanden speichert.

6. Zugangsvorrichtung (4) nach einem der Ansprüche 1 bis 5, wobei die Zugangssteuerstufe (18) so eingerichtet ist, dass sie das Erkennungssignal empfängt,
wobei die Zugangssteuerstufe (18) ferner so eingerichtet ist, dass sie mit einer externen Datenverarbeitungseinheit (6) eine vorzugsweise drahtlose Verbindung (20) herstellt und das von dem Sensor (10) empfangene Erkennungssignal über die drahtlose Verbindung (20) an die Datenverarbeitungseinheit (6) überträgt,
wobei die Zugangssteuerstufe (18) ferner so eingerichtet ist, dass sie von der Datenverarbeitungseinheit (6) über die drahtlose Verbindung (20) ein Analysesignal empfängt, das aus dem Erkennungssignal erstellt wird und die Zugangsinformationen umfasst.

7. Zugangsvorrichtung (4) nach Anspruch 6, wobei die Zugangssteuerstufe (18) auch so eingerichtet ist, dass sie Energie über die drahtlose Verbindung (20) empfängt, wobei die Zugangssteuerstufe so eingerichtet ist, dass sie mindestens einen Teil der empfangenen Energie entnimmt, um ein Abfragesignal des Sensors (10) zu erzeugen, wobei der Sensor (10) so eingerichtet ist, dass er das Erkennungssignal nach dem Empfang des von der Zugangssteuerstufe (18) erzeugten Abfragesignals ausgibt.

8. Zugangsvorrichtung (4) nach einem der Ansprüche 1 bis 7, wobei die Zugangssteuerstufe (18) mittels eines Verbindungsbusses (14) elektrisch mit dem Sensor (10) verbunden ist, wobei der Aufbewahrungsteil (26) der Halterung (8) so eingerichtet ist, dass er von dem Einwegteil (24) durch Brechen entlang einer vorbestimmten Bruchlinie (28) getrennt wird, wobei der Verbindungsbus (14) so eingerichtet ist, dass er sich bei dem Trennen des Aufbewahrungsteils (26) von dem Einwegteil (24) vorzugsweise an der Bruchlinie (28) trennt.

9. Zugangsvorrichtung (4) nach einem der Ansprüche 1 bis 7, wobei die Zugangssteuerstufe (18) mittels eines Verbindungsbusses (14) elektrisch mit dem Sensor (10) verbunden ist, wobei der Aufbewahrungsteil (26) der Halterung (8) so eingerichtet ist, dass er durch Ablösung an einem vorbestimmten Klebebereich (30) von dem Einwegteil (24) getrennt wird, wobei der elektrische Durchgang des Verbindungsbusses (14) in dem Klebebereich (30) durch einen leitenden Klebstoff oder durch Vias gewährleistet ist, die so eingerichtet sind, dass sie bei dem Ablösen brechen.

10. Zugangsvorrichtung (4) nach einem der Ansprüche 1 bis 7, wobei die Zugangssteuerstufe (18) mittels eines Verbindungsbusses (14) elektrisch mit dem Sensor (10) verbunden ist, der Aufbewahrungsteil (26) der Halterung (8) durch einen Mehrfachkontaktverbinder (50) mit dem Einwegteil (24) verbunden ist, wie beispielsweise ein USB-Schlüsselsystem oder eine Speicherkarte, und der Aufbewahrungsteil (26) so eingerichtet ist, dass er durch Trennen von dem Einwegteil (24) gelöst wird, wobei der Verbindungsbus (14) so eingerichtet ist, dass er unterbrochen wird, vorzugsweise im Bereich der Trennstelle, wenn der Aufbewahrungsteil (26) von dem Einwegteil (24) getrennt wird, wobei ein Steckerteil des Verbinders (50) entweder am Aufbewahrungsteil (26) oder am Einwegteil (24) angebracht sein kann.

11. Zugangsvorrichtung (4) nach einem der Ansprüche 1 bis 10, wobei die Zugangssteuerstufe (18) mindestens einen Teil eines Funketiketts bildet, das vorzugsweise zur Durchführung einer Nahfeldkommunikation geeignet ist.

12. Zugangsvorrichtung (4) nach einem der Ansprüche 1 bis 11, wobei der Sensor (10) und der Aufbewahrungsteil (26) der Halterung (8) voneinander entfernt sind, wobei ein Abstand zwischen dem Sensor (10) und dem Aufbewahrungsteil (26) der Halterung (8) größer ist als eine vorbestimmte Kontaminationsgrenze.

13. Zugangsschlüsselerzeugungssystem (2), umfassend die Zugangsvorrichtung (4) nach einem der Ansprüche 1 bis 12 und eine Datenverarbeitungseinheit (6), die so eingerichtet ist, dass sie das Erkennungssignal empfängt, aus dem empfangenen Erkennungssignal ein Analysesignal erzeugt, das die Zugangsinformationen umfasst, und das Analysesignal an die Zugangssteuerstufe (18) bereitstellt.

14. Zugangssteuerverfahren zum Zulassen oder Nichtzulassen des Zugangs eines Probanden zu einem Zugangsbeschränkungsbereich, wobei das Verfahren die Zugangsvorrichtung (4) nach einem der Ansprüche 1 bis 13 implementiert und folgende Schritte umfasst:
- Messen, mittels des Sensors (10), mindestens eines Gesundheitsparameters des Probanden;
- Generieren der Zugangsinformationen in Abhängigkeit von dem Erkennungssignal;
- Speichern der Zugangsinformationen in der Zugangssteuerstufe (18); und
- Trennen des Aufbewahrungsteils (26) der Halterung (8) von dem Einwegteil (24), um den Zugangsschlüssel zu bilden, vorzugsweise ferner umfassend eine Registrierung einer eindeutigen Kennung des Probanden in der Zugangssteuerstufe (18).

15. Zugangssteuerverfahren nach Anspruch 14, ferner umfassend:
- Übertragen des von dem Sensor (10) gelieferten Erkennungssignals an eine Datenverarbeitungseinheit (6) über eine drahtlose Verbindung (20), vorzugsweise verschlüsselt;
- Analysieren des von dem Sensor (10) gelieferten Erkennungssignals durch die Datenverarbeitungseinheit (6), um einen Wert für jeden Gesundheitsparameter zu bestimmen, und Erzeugen der Zugangsinformationen in Abhängigkeit von dem Analysieren des Erkennungssignals; und
- nach dem Erzeugen der Zugangsinformationen durch die Datenverarbeitungseinheit (6), Übertragen eines Analysesignals, das die erzeugten Zugangsinformationen umfasst, von der Datenverarbeitungseinheit (6) über die drahtlose Verbindung an die Zugangssteuerstufe (18).

16. Zugangssteuerverfahren nach einem der Ansprüche 14 oder 15, ferner umfassend folgende Schritte:
- Vorlegen des gebildeten Zugangsschlüssels an einem Lesegerät, das einen Eingang des Zugangsbeschränkungsbereichs ausstattet;
- Lesen der in der Zugangssteuerstufe (18) des Zugangsschlüssels gespeicherten Zugangsinformationen mittels des Lesegeräts;
- Zulassen oder Nichtzulassen des Zugangs des Probanden zu dem Beschränkungsbereich in Abhängigkeit von den gelesenen Zugangsinformationen, wobei vorzugsweise während des Zulassens oder Nichtzulassens zu dem Zugangsbeschränkungsbereich der Zugang zu dem Zugangsbeschränkungsbereich zugelassen wird, wenn die Zugangsinformationen das Fehlen mindestens eines vorbestimmten Analyten in einer biologischen Probe angeben, die von dem Probanden entnommen wurde, oder wenn ein Wert eines Gesundheitsparameters zu einem vorbestimmten Intervall gehört, und andernfalls verweigert wird.

## Claims

1. A portable device (4) for accessing an area with restricted access, the access device is single-use and comprises a support (8), a sensor (10) and an access control stage (18),
the portable access device is single-use in that the sensor (10) is intended to be placed in contact with a biological sample taken from a subject,
the support (8) comprising a portion (24) to be discarded and a portion (26) to be retained,
the sensor (10) being borne by the portion (24) to be discarded of the support (8) and being configured to measure at least one health parameter of a subject, and to deliver a detection signal representative of the measurement of each health parameter,
the access control stage (18) being borne by the portion (26) to be retained of the support (8) and being configured to store, preferably in encrypted form, access information dependent on the detection signal delivered by the sensor (10),
the portion (26) to be retained of the support (8) being able to be mechanically separated from the portion (24) to be discarded so as to form an access key allowing or prohibiting, according to the access information, access to the area with restricted access.

2. The access device (4) according to claim 1, wherein the sensor (10) is configured to deliver a detection signal representative of the presence or absence of at least one predetermined analyte in the biological sample, the detection signal also being representative of the presence or absence of at least one predetermined analyte in the biological sample.

3. The access device (4) according to claim 1 or 2, wherein the sensor (10) is composed of a graphene sheet, said graphene sheet being covered with a thin layer having specific functional groups of a given analyte.

4. The access device (4) according to claim 1, 2 or 3, wherein the access information comprises the detection signal and/or data relating to the presence or absence of at least one predetermined analyte in a biological sample taken from the subject and/or data relating to a time stamp and to a period of validity of an access authorisation and/or data relating to a location of validity of the access authorisation and, preferably, a unique identifier of the subject.

5. The access device (4) according to any one of claims 1 to 4, wherein the access control stage (18) is further configured to store a unique identifier of the subject.

6. The access device (4) according to any one of claims 1 to 5, wherein the access control stage (18) is configured to receive the detection signal,
the access control stage (18) being further configured to establish, with an external data processing unit (6), a wireless link (20), preferably encrypted, and to transmit, to the data processing unit (6), via the wireless link (20), the detection signal received from the sensor (10),
the access control stage (18) also being configured to receive, from the data processing unit (6), via the wireless link (20), an analysis signal established based on the detection signal and comprising the access information.

7. The access device (4) according to claim 6, wherein the access control stage (18) is also configured to receive energy via the wireless link (20), the access control stage being configured to draw at least a portion of the received energy in order to generate an interrogation signal of the sensor (10), the sensor (10) being configured to deliver the detection signal following the reception of the interrogation signal generated by the access control stage (18).

8. The access device (4) according to any one of claims 1 to 7, wherein the access control stage (18) is electrically connected to the sensor (10) by means of a connection bus (14), the portion (26) to be retained of the support (8) being configured to be separated from the portion (24) to be discarded by being broken along a predetermined fracture line (28), the connection bus (14) being configured to break, preferably at the fracture line (28), upon separation of the portion (26) to be retained from the portion (24) to be discarded.

9. The access device (4) according to any one of claims 1 to 7, wherein the access control stage (18) is electrically connected to the sensor (10) by means of a connection bus (14), the portion (26) to be retained of the support (8) being configured to be separated from the portion (24) to be discarded by detachment at a predetermined bonding zone (30), the electrical continuity of the connection bus (14) in the bonding zone (30) being ensured by a conductive adhesive or by vias configured to break upon detachment.

10. The access device (4) according to any one of claims 1 to 7, wherein the access control stage (18) is electrically connected to the sensor (10) by means of a connection bus (14), the portion (26) to be retained of the support (8) is connected to the portion (24) to be discarded by a multi-contact connector (50), such as for example a USB key system or a memory card, and the portion (26) to be retained is configured to be separated from the portion (24) to be discarded by disconnection, the connection bus (14) being configured to be discontinued, preferably at the disconnection zone, upon separation of the portion (26) to be retained from the portion (24) to be discarded, a male portion of the connector (50) being able to be contained either on the portion (26) to be retained or on the portion (24) to be discarded.

11. The access device (4) according to any one of claims 1 to 10, wherein the access control stage (18) forms at least one portion of a radio tag, preferably able to implement near-field communication.

12. The access device (4) according to any one of claims 1 to 11, wherein the sensor (10) and the portion (26) to be retained of the support (8) are spaced apart from each other, a distance between the sensor (10) and the portion (26) to be retained of the support (8) being greater than a predetermined contamination limit.

13. An access key generation system (2) comprising the access device (4) according to any one of claims 1 to 12, and a data processing unit (6) configured to receive the detection signal, to generate, based on the detection signal received, an analysis signal comprising the access information, and to provide the analysis signal to the access control stage (18).

14. An access control method for authorising or not authorising the access of a subject to an area with restricted access, the method implementing the access device (4) according to any one of claims 1 to 13 and comprising the steps of:
- measuring, by means of the sensor (10), at least one health parameter of the subject;
- generating the access information according to the detection signal;
- storing the access information in the access control stage (18); and
- separating the portion (26) to be retained of the support (8) from the portion (24) to be discarded so as to form the access key, preferably further comprising saving, in the access control stage (18), a unique identifier of the subject.

15. The access control method according to claim 14, further comprising:
- transmitting the detection signal delivered by the sensor (10) to a data processing unit (6), via a wireless link (20), preferably encrypted;
- analysing, by the data processing unit (6), the detection signal delivered by the sensor (10), to determine a value of each health parameter, and generating the access information according to the analysis of the detection signal; and
- after the access information has been generated by the data processing unit (6), transmitting to the access control stage (18) an analysis signal comprising the access information generated, from the data processing unit (6), via the wireless link.

16. The access control method according to claim 14 or 15, further comprising the steps of:
- presenting the access key formed to a reader with which an entrance of the area with restricted access is equipped;
- reading, by means of the reader, the access information stored in the access control stage (18) of the access key;
- authorising or not authorising the access of the subject to the area with restricted access according to the access information read, preferably wherein, during the authorisation or non-authorisation of the access to the area with restricted access, the access to the area with restricted access is authorised if the access information indicates the absence of at least one predetermined analyte in a biological sample taken from the subject or if a value of a health parameter is within a predetermined interval, and is prohibited otherwise.
